Europäisches Patentamt

European Patent Office

Office européen des brevets

(11) Publication number: **0 299 342**
**A1**

## EUROPEAN PATENT APPLICATION

(21) Application number: 88110756.9

(22) Date of filing: 06.07.88

(51) Int. Cl.⁴: **C04B 35/48 , A61C 13/083 , A61K 6/02 , A61L 27/00 , A61C 8/00**

(30) Priority: 10.07.87 JP 173524/87

(43) Date of publication of application:
**18.01.89 Bulletin 89/03**

(84) Designated Contracting States:
**AT BE CH DE ES FR GB GR IT LI LU NL SE**

(71) Applicant: Takeda Chemical Industries, Ltd.
27, Doshomachi 2-chome Higashi-ku
Osaka-shi Osaka, 541(JP)

Applicant: Agency of Industrial Science and
Technology of Ministry of International
Trade and Industry
3-1, Kasumigaseki 1-chome
Chiyoda-ku Tokyo(JP)

(72) Inventor: Tamari, Nobuyuki
12-102, 11 Kasugacho 5-chome
Toyonaka Osaka 560(JP)
Inventor: Mouri, Motoya
5-122, Higashiohda 1-chome
Ibaraki Osaka 567(JP)

(74) Representative: von Kreisler, Alek,
Dipl.-Chem. et al
Patentanwälte Von Kreisler-Selting-Werner
Deichmannhaus am Hauptbahnhof
D-5000 Köln 1(DE)

(54) **Bioceramic materials.**

(57) Bioceramic materials obtained by sintering a mixture of zirconia, a calcium phosphate compound and a metal fluoride, assure improved adhesion to biological tissues, the quality of utmost importance in any bioceramic material and contribute to improved processablility.

EP 0 299 342 A1

## Bioceramic Materials

The present invention relates to bioceramic materials which can be used in such applications as artificial bones, artificial dental roots and so on.

The bioceramic materials available on the market today are based on alumina or hydroxyapatite. Alumina excels in mechanical strength but, being biologically inert, has only a poor affinity for biological tissues.

On the other hand, calcium phosphate compounds such as hydroxyapatite consist of the same inorganic elements as natural bones and are therefore biologically active to assure excellent bone adhesion and biocompatibility. However, these compounds are fairly inferior to alumina in mechanical properties, being particularly low in bending strength and toughness, so that they are not fully satisfactory for bioceramic applications. Recently, under these circumstances, research has been in progress for the development of a composite material consisting of zirconia, which is superior in mechanical properties but is biologically inert, and calcium phosphate compounds, which are inferior in mechanical properties but are superior in biocompatibility [Toku-kai Sho 62-142565].

This composite material has both strength and biocompatibility in one but because of incorporation of biologically inert zirconia, it is naturally inferior to the single-component calcium phosphyate material in biocompatibility. Therefore, the development of a composite material having improved mechanical properties with a reduced zirconia content has been demanded.

It is an object of the invention to provide a bioceramic material which excels in biocompatibility as well as in physical characteristics inclusive of mechanical properties and fracture toughness.

The present inventors explored the technology for the production of composite materials based on zirconia and calcium phosphate compounds and particularyl a bioceramic material with a relatively high proportion of highly biocompatible calcium phosphate compounds and yet showing a high degree of mechanical properties and found that the addition of metal fluorides at a low level to such a composite system permits increasing the proportion of calcium phosphate component without loss of mechanical properties. The present invention is the outgrowth of the above research finding. The present invention is therefore directed to a bioceramic material obtained by sintering a mixture of zirconia, a calcium phosphate compound and a metal fluoride.

Zirconia, as used in accordance with the invention, may be a commercial preparation but from the standpoint of strength and toughness, one partially stabilized with yttria, magnesia, ceria or the like is preferred to stabilized zirconia. From the standpoint of sinterability, strength and so on, the particle size of zirconia is preferably as small as possible and most desirably not larger than about 1 μm.

The calcium phosphate compounds to be used according to the invention include hydroxyapatite, tricalcium phosphate, tetracalcium phosphate, octacalcium phosphate and so on, and these compounds can be used singly or in combination. The proportions of such different species of calcium phosphate compunds are virtually optional and can be selected as desired. The calcium phosphate compounds mentioned above may be commercial products and there is no particular limitation on the production method involved, particle size, purity or the like. However, in view of uses for a bioceramic material, these compounds are preferably of high purity and from the standpoint of sinterability and mechanical strength, those in a size range not exceeding about 1 μm are preferably used.

The metal fluorides used according to the invention are exemplified by fluorides of monovalent metals such as sodium fluoride, potassium fluoride, etc.; fluorides of divalent metals such as magnesium fluoride, calcium fluoride, etc.; fluorides of trivalent metals such as aluminum fluoride; fluorides of tetravalent metals such as titanium fluoride etc.; and other metal fluorides such as potassium titanium fluoride and so on. Particularly preferred are calcium fluoride and magnesium fluoride. In order that these metal fluorides may be uniformly blended with said calcium phosphate compounds are zirconia, their particle sizes are preferably as small as possible, desirably not over about 1 μm.

The preferred ratio of zirconia to calcium phosphate compounds is such that zirconia accounts for about 15 to 75 percent by volume and, for still better results, about 30 to 60 percent by volume. If the proportion of zirconia is less than 15 percent by volume, the strength and toughness of the resulting sintered body will not be much different from those of the product based on calcium phosphate compounds alone.

On the other hand, if the proportion of zirconia exceeds 75 percent by volume, there will be realized improvements in strength and toughness but because of deficiencies in calcium phosphate content, the product may show only poor adhesion to biological tissues.

The proportion of said metal fluoride is preferably about 0.1 to 1.5 percent as fluorine and more desirably about 0.25 to 1.0 percent as fluorine based on the combined weight of calcium phosphate compounds and zirconia.

In accordance with the present invention, zirconia, calcium phosphate compounds and metal fluoride are blended in the first place. This blending may be accomplished any technique applicable to blending of particulate or granular materials, such as dry mixing in a mixer or wet mixing in a pot mill.

The ternary composition thus obtained is dried, if necessary, and then molded and sintered. The molding technique that can be used includes slurry casting, extrusion, injection molding, cold isostatic pressing, hot pressing, hot isostatic pressing, and so on.

The sintering technique may be any of normal sintering, pressure sintering, special atmospheric sintering and so on. The sintering temperature is dependent on the proportions and species of said three component materials, molding conditions, etc., but is preferably in the range of about 1,100 to 1,500°C for all practical purposes. If the sintering temperature is below 1100°C, there may not be obtained a sufficiently compact body and the mechanical properties of the product may not be sufficiently high to warrant its use as a bioceramic material.

If the sintering temperature is more than 1,500°C, there may occur an abnormal growth of constituent particles to produce coarse grains detracting from the strength of the sintered body and from its adhesivity to biological tissues. The sintering time is, for example, about 10 to 30 minutes in the case of hot pressing and about 30 to 120 minutes for normal sintering.

If the sintering time is too short, no sufficient compactness will be attained, while excessively prolonged sintering may give rise to a texture pf coarse grains.

In accordance with the invention, bioceramic materials with improved mechanical properties can be provided by a simple process comprising adding a small amount of metal fluoride to a composite of zirconia and calcium phosphate compounds and sintering the resulting composition. The addition of metal fluoride permits decreasing the proportion of zirconia, assures improved adhesion to biological tissues, the quality of utmost importance in any bioceramic material, and contributes to improved processability. The following working and comparative examples are further illustrative of the invention.

Examples 1 to 4

Partially stabilized zirconia powder (manufactured by Toyo Soda Mfg. Co., Ltd., containing 2 mol % of yttria, particle size 0.2-0.3 $\mu$m, specific surface area 14-23 $m^2$/g) and hydroxyapatite powder (manufactured by Central Glass Co., Ltd.; Type AN, particle size $\leq$ 0.1 $\mu$m, specific surface area 74 $m^2$/g) were blended in a ratio of 50:50 by volume, followed by addition of calcium fluoride (manufactured by Wako Pure Chemicals Co., Ltd.; special reagent grade) at the levels indicated an Table 1. The mixture was wet-mixed in a pot mill for 24 hours and dried.

The mixed powder thus obtained was hot-pressed at 1,350°C and a pressure of 200 kg/cm$^2$ for 10 minutes. The relative density of this sinterd body was determined by the method of Archimedes and testpieces measuring 3 mm x 3 mm x 30 mm were cut out and subjected to the three-point bending test under the conditions of 20 mm span and 0.5 mm/min. loading speed. In addition, one side of each testpiece was polished mirror smooth with a diamond paste and determined for fracture toughness under a load of 5 kg in accordance with the IF (indentation fracure) test method. The results of these tests are shown in Table 1.

Comparative Example 1

Using the same partially stabilized zirconia and hydroxyapatite as used in Examples 1 to 4 but not using calcium fluoride, sintered testpieces were prepared under otherwise the same conditions as Examples. Each of these tespieces was tested for bending strength, fracture toughness and relative density in the same manner as Examples. The results are shown, alongside data on Examples, in Table 1.

Table 1

| | Mixing ratio | | Physical values of bioceramic materials | | |
|---|---|---|---|---|---|
| | Zirconia/hydroxyapatite, V/V | Level of calcium fluoride (as F) (%) | Relative density (%) | Bending strength (MPa) | Fracture toughness (MPa • $\sqrt{m}$) |
| Example 1 | 50/50 | 0.24 | 97.0 | 380 | 2.7 |
| Example 2 | 50/50 | 0.42 | 98.3 | 470 | 2.8 |
| Example 3 | 50/50 | 0.73 | 98.5 | 455 | 2.8 |
| Example 4 | 50/50 | 1.22 | 97.8 | 320 | 2.5 |
| Comparative Example 1 | 50/50 | 0 | 94.5 | 270 | 2.4 |

Example 5

Using the same materials as those mentioned in Examples 1 to 4 but calcium fluoride at the level of 0.5 weight % (0.24% as fluorine), test pieces sintered at 1,400°C were prepared and determined for various physical properties. The results are shown in Table 2.

Table 2

|  | Sintering temperature | Relative density (%) | Bending strength (MPa) | Fracture toughess (MPa • √m) |
|---|---|---|---|---|
| Example 5 | 1,400°C | 98.1 | 460 | 3.2 |

Examples 6 to 7

Using the same materials as those used in Example 1 except that the mixing ratio of partially stabilized zirconia powder to hydroxyapatite was adjusted to 40/60 or 30/70, test pieces were prepared and sintered at 1,350°C in the same manner as Example 1. These test pieces were determined for various physical properties by the same procedures as described in Example 1. The results are shown in Table 3.

Table 3

|  | Mixing ratio | Physical values of bioceramic materials | | |
|---|---|---|---|---|
|  | Zirconia/hydroxyapatite,v:v | Relative density (%) | Bending strength (MPa) | Fracture toughness (MPa • √m) |
| Example 6 | 40/60 | 98.8 | 410 | 2.7 |
| Example 7 | 30/70 | 99.2 | 328 | 2.2 |
| Comparative Example 1 | 50/50 | 94.5 | 270 | 2.4 |

It is clear from Table 3 that, compared with Comparative Example 1, there is no loss of mechanical strength despite a decreased proportion of zirconia, thus having enabled one to increase the proportion of hydroxyapatite which is contributory to biocompatibility.

**Claims**

1. A bioceramic material obtained by sintering a mixture of zirconia, a calcium phosphate compound and a metal fluoride.

2. A bioceramic material claimed in claim 1, wherein zirconia is a stabilized zirconia with yttria.

3. A bioceramic material claimed in claim 1, wherein the calcium phophate compound is hydroxyapatite.

4. A bioceramic material claimed in claim 1, wherein the metal fluoride is calcium fluoride.

5. A bioceramic material claimed in claim 1, wherein the ratio of zirconia to calcium phosphate compound is such that zirconia accounts for about 15 to 75 percent by volume.

6. A bicoceramic material claimed in claim 1, wherein the proportion of metal fluoride is about 0.1 to 1.5 percent.

7. A bioceramic material claimed in claim 1, wherein the sintering temperature is in the range of about 1,100 to 1,500°C.

8. A process for producing a bioceramic material which comprises sintering a mixture of zirconia, a calcium phosphate compound and a metal fluoride.

| Category | Citation of document with indication, where appropriate, of relevant passages | Relevant to claim | CLASSIFICATION OF THE APPLICATION (Int. Cl.4) |
|---|---|---|---|
| | **DOCUMENTS CONSIDERED TO BE RELEVANT** | | EP 88110756.9 |
| D,A | JP - A2 - 62-142 565 (AGENCY OF INDUSTRIAL SCIENCE & TECHNOLOGY) <br> * Totality * <br><br> -- | 1-8 | C 04 B 35/48 <br> A 61 C 13/083 <br> A 61 K 6/02 <br> A 61 L 27/00 |
| A | US - A - 4 626 392 (KONDO et al.) <br> * Totality * <br><br> ---- | 1-8 | A 61 C 8/00 |
| | | | **TECHNICAL FIELDS SEARCHED (Int. Cl.4)** <br><br> C 04 B <br> A 61 C <br> A 61 K <br> A 61 L |

The present search report has been drawn up for all claims

| Place of search | Date of completion of the search | Examiner |
|---|---|---|
| VIENNA | 23-09-1988 | BECK |